(19) Europäisches Patentamt
European Patent Office
Office européen des brevets

(11) Publication number: 0 141 057 B1

(12) # EUROPEAN PATENT SPECIFICATION

(45) Date of publication of patent specification: 31.08.88

(51) Int. Cl.⁴: C 07 H 17/04, A 61 K 31/70

(21) Application number: 84108834.7

(22) Date of filing: 26.07.84

(54) **Novel 4'-demethyl-4-epipodophyllotoxin derivatives, a process for their preparation and their use as medicaments.**

(30) Priority: 29.07.83 JP 137687/83

(43) Date of publication of application: 15.05.85 Bulletin 85/20

(45) Publication of the grant of the patent: 31.08.88 Bulletin 88/35

(84) Designated Contracting States: AT BE CH DE FR GB IT LI LU NL SE

(58) References cited:
US-A-3 524 844

CHEM. ABST. REG. HALDB., 1980, page 953RI; Anticancer Agents based on Natural Product Models, Academic Press (1980), pages 319-351

(73) Proprietor: MICROBIAL CHEMISTRY RESEARCH FOUNDATION
14-23, Kamiosaki 3 Chome Shinagawa-ku
Tokyo 141 (JP)

(72) Inventor: Umezawa, Hamao, Prof.
4-23, Toyotama-kita Nerimaku
Tokyo (JP)
Inventor: Takeuchi, Tomio
5-1-11, Higashi-Gotanda Shinagawa-ku
Tokyo (JP)
Inventor: Kondo, Shinichi
1157-1, Ichigao-cho Midori-ku
Yokohama-city Kanagawa Prefecture (JP)
Inventor: Tanaka, Wataru
9-20, Hibarigaoka 1-chome
Hoya-city Tokyo (JP)
Inventor: Takita, Tomohisa
7-10-15, Negishidai
Asaka-city Saitama Prefecture (JP)
Inventor: Nishimura, Yoshio
30-1-333, Baba 7-chome Tsurumi-ku
Yokohama-City Kanagawa Prefecture (JP)
Inventor: Yoshikawa, Hiroshi
326-2, Shimokurisu
Fujioka-city Gumma Prefecture (JP)

EP 0 141 057 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(74) Representative: **Becker, Heinrich Karl Engelbert, Dr. et al**
**HOECHST AKTIENGESELLSCHAFT**
**Central Patent Department**
**P.O. Box 80 03 20**
**D-6230 Frankfurt am Main 80 (DE)**

**Description**

The present invention relates to novel 4'-demethyl-4-epipodophyllotoxin derivatives of the general formula I

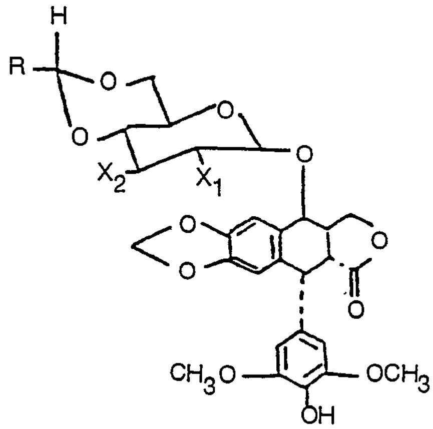

wherein R represents a $C_1$—$C_4$ alkyl group, $X_1$ represents a hydroxyl group or an amino group, and $X_2$ represents an amino group or a hydroxy group, with the proviso that when one of $X_1$ and $X_2$ represents an amino group, the other represents a hydroxy group, and when one of them represents a hydroxy group, the other represents an amino group, and a salt thereof.

So far, 4'-demethyl-4-epipodophyllotoxin-β-D-alkylideneglycosides have been known from US—A—3 524 844 and Japanese Patent Publication No. 38258/70 as compounds having antitumor activity.

It was further known from "Anticancer Agents based on Natural Product Models" (Academic Press, 1980), pages 319—351, in particular pages 344—346, that variations in the structure of and around the sugar moiety, for example the substitution of galactose for glucose or even small changes in the substituents of the glucopyranose moiety, may have a strong influence on bioactivity.

Now, compounds of the aforementioned general formula (I) have been found which show excellent antitumor activity.

A 4'-demethyl-4-epipodophyllotoxin derivative of the general formula (I) according to this invention is obtained in the following way:

4'-O-Protected-4'-demethyl-4-epipodophyllotoxin of the general formula (II)

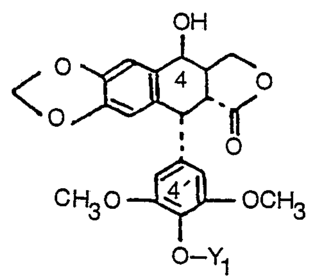

(II)

wherein $Y_1$ represents a protective group, is reacted with a compound of the general formula (III)

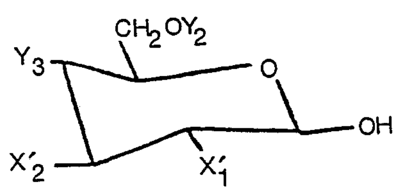

(III)

wherein $Y_2$ and $Y_3$ each represents a protective group, and one of $X'_1$ and $X'_2$ represents a protected amino group and the other represents a protected hydroxy group, in an inert solvent in the presence of a condensation catalyst, such as boron trifluoride etherate to form a compound of the general formula (IV)

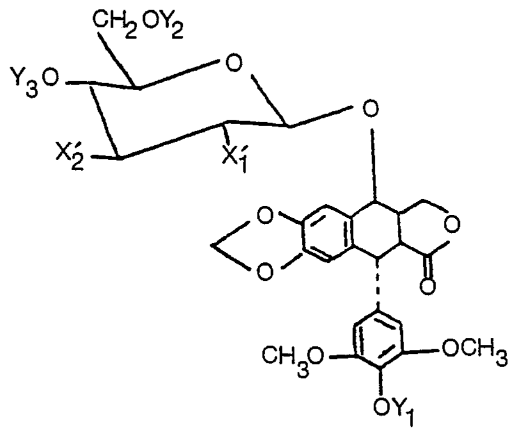

(IV)

wherein $Y_1$, $Y_2$, $Y_3$, $X'_1$ and $X'_2$ are as defined above. This compound is subjected to a step for removing the protective groups $Y_2$ and $Y_3$ therefrom (the protective group for hydroxyl may be removed from $X'_1$ or $X'_2$ by this step) to form a compound of the general formula (V)

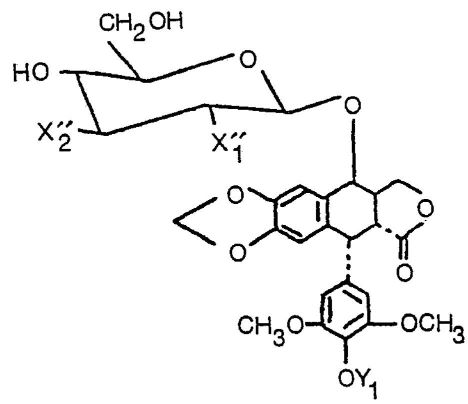

(V)

wherein one of $X''_1$ and $X''_2$ represents a hydroxyl group or a protected hydroxyl group and the other represents a protected amino group, and $Y_1$ is as defined above.

The resulting compound is condensed with an aldehyde of the general formula (VI)

RCHO (VI)

wherein R is as defined previously, or its acetal compound in the presence of a condensing agent, such as p-toluenesulfonic acid, to form a compound of the general formula (VII)

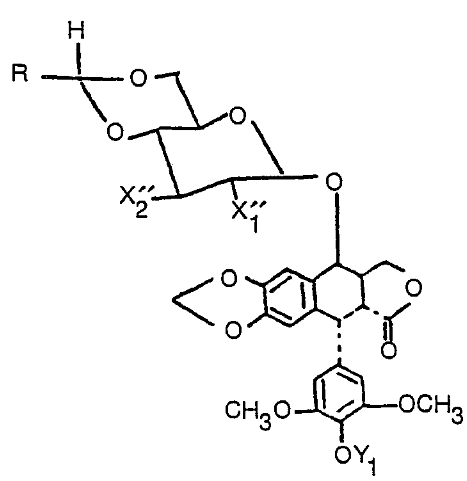

(VII)

wherein R, $X''_1$, $X''_2$ and $Y_1$ are each as defined above.

The resulting compound is subjected to a step for removing the protective groups therefrom to obtain a novel 4'-demethyl-4-epipodophyllotoxin derivative of the general formula (I).

$C_1$—$C_4$ alkyl as R in the present invention means particularly methyl, ethyl or propyl. Methyl is particularly preferred.

The protective group for amino and the protective group for hydroxyl in this invention are not limited to particular groups, while it is necessary that $Y_2$ and $Y_3$ be protective groups which can be removed by different treating means from those used for $Y_1$ and the protective group for amino. Assume that in case $Y_1$ and the protective group for amino are those which will be removed by catalytic reduction using palladium and platinum catalysts, for example, benzyloxycarbonyl optionally having a substituent; the protective groups $Y_2$ and $Y_3$ should preferably be those which can be removed by different treating means, such as hydrolysis, acid decomposition, or ester interchange using zinc acetate. Examples of such latter protective groups are lower alkylcarbonyl groups, such as acetyl. The protective group for hydroxyl in $X'_1$ or $X'_2$ may be any group which is commonly used as a protective group.

The compound of this invention can form salts with acids by customary methods. Examples of acids for forming such salts are inorganic and organic acids, such as hydrochloric acid, sulfuric acid, phosphoric acid, acetic acid and citric acid.

Typical compounds of this invention are for example the following:

(1) 4-O-(2-Amino-2-deoxy-4,6-ethylidene-β-D-glucopyranosyl)-4'-demethyl-4-epipodophyllotoxin (Compound No. 1)

(2) 4-O-(3-Amino-3-deoxy-4,6-O-ethyliden-β-D-glucopyranosyl)-4'-demethyl-4-epipodophyllotoxin (Compound No. 2)

These compounds have very potent antitumor activity.

Determination of solubility of etoposide derivatives ''4-O-(2-amino-4,6-ethylidene-2-deoxy-β-D-glucopyranosyl)-4'-demethyl-4-epipodophyllotoxin

*Method*

11.75 mg of 4-O-(2-amino-4,6-ethylidene-2-deoxy-β-D-glucopyranosyl)-4'-demethyl-4-epipodophyllotoxin was taken into a test tube, and 0.5 ml of distilled water was added. The mixture was stored at 25°C with occasional shaking. After 6 and 29 hours, 50 µl each of samples was taken from the mixture, and filtered. 30 µl of the filtrate was diluted with 4 ml of water, and the dilution was measured for absorbance at 285 nm.

The concentration of the compound was calculated with $E^{1\%}_{1\,cm}$ (285 nm, $H_2O$) determined to be 59.6.

*Results*

| | OD.285 | Conc. |
|---|---|---|
| 6 hrs | 62.03 | 10.4 mg/ml |
| 29 hrs | 64.08 | 10.7 mg/ml |

The solubility of compound No. 15 in water was thus found to be 10 mg/ml (1%). [A similar test showed the water-solubility of VP—16 to be 0.1 mg/ml (0.01%)]

Tests on antitumor activity against murine leukemia L1210

a) Six-week-old female $CDF_1$ mice were inoculated with $1 \times 10^3$ cells of L1210, and the test drug was administered intraperitoneally once daily for 5 days, starting on the day after inoculation. For the group of mice receiving no treatment with that drug (non-treatment group), physiological saline solution was similarly administered. After initiation of the administration, the animals were observed for death for 60 days. The average survival period in days for each of the treatment and non-treatment groups was determined, and the results were designated as T and C, respectively. (T/C) × 100 (%) was calculated from the results.

| Dose (mg/kg) | T/C (%) | Average survival period (days) (mean + S.D.) | Surviving mice * |
|---|---|---|---|
| 10.0 | 597 | 43.6 ± 23.5 | 3/6 |
| 5.0 | 395 | 28.8 ± 19.4 | 1/5 |
| 2.5 | 184 | 13.4 ± 2.2 | 0/5 |
| 1.25 | 159 | 11.6 ± 1.9 | 0/5 |
| 0.625 | 167 | 12.2 ± 1.5 | 0/5 |
| 0.313 | 137 | 10.0 ± 0.7 | 0/5 |

* Number of mice surviving for 60 days / Number of total mice per group

b) 105 murine leukemia L1210 cells were inoculated intraperitoneally into mice, and a suspension of the compound of this invention in physiological saline solution was administered intraperitoneally once daily for 9 consecutive days, starting 24 hours after the inoculation. The animals were observed for 41 days, and the increase of life span (ILS) in these animals was calculated from the following equation:

$$ILS = \frac{\text{Average period (in days) of survival in the group receiving the compound of this invention}}{\text{Average period (in days) of survival in the control group}} \times 100$$

The control group was administered with physiological saline solution only. The average survival period for the control group was 7.9 to 8.3 days.

Compound No. 1 in a dose of 100 µg/kg/day was found to give ILS of 494 or more, and Compound No. 2 in a dose of 25 µg/kg/day, ILS of 430 and more.

These results show that the compounds of this invention exhibit excellent antitumor effect.

The methods for synthesis of the compound according to this invention will be described in greater detail with reference to the following Examples:

Example 1

(1) Synthesis of 4-O-(2-benzyloxycarbonylamino-2-deoxy-β-D-glucopyranosyl)-4′-benzyloxycarbonyl-4′-demethyl-4-epipodophyllotoxin

(a) 500 mg of 4′-benzyloxycarbonyl-4′-demethyl-4-epipodophyllotoxin and 620 mg of 3,4,6-tri-O-acetyl-2-benzyloxycarbonyl-amino-2-deoxy-β-D-glucopyranose were dissolved in 1 ml of dichloromethane. To the resulted solution cooled to −18°C, 0.5 ml of $BF_3 \cdot Et_2O$ was added dropwise over 3 minutes, and the mixture reacted for 30 minutes under the atmosphere of argon. 0.5 ml of pyridine was then added to terminate the reaction, and the reaction mixture was diluted with 20 ml of dichloromethane. The organic layer was washed twice with 10 ml of water, dried over Glauber's salt, and concentrated. The concentrate was subjected to silica gel chromatography for separation and purification. 700 mg of 4 - O - (3,4,6 - tri - O - acetyl - 2 - benzoyloxy - carbonylamino - 2 - deoxy - β - D - glucopyranosyl) - 4′ - benzyloxycarbonyl - 4′ - demethyl - 4 - epipodophyllotoxin was obtained.

Specific rotation: $[\alpha]_D^{21} = -39.6°$ ($CHCl_3$)

(b) 600 mg of the compound obtained in the above step and 115 mg of zinc acetate were dissolved in 5 ml of methanol, and the solution was boiled for 6 hours under reflux to cause the reaction. The reaction mixture was evaporated to dryness, and 20 ml of dichloromethane and 10 ml of water were added to the residue. The mixture was shaken vigorously to separate the organic layer. The separated layer was dried over Glauber's salt and concentrated. The concentrate was subjected to silica gel chromatography for separation and purification. 295 mg of 4-O-(2-benzoyloxycarbonyl-amino-2-deoxy-β-D-glucopyranosyl)-4′-benzyloxycarbonyl-4′-demethyl-4-epipodophyllotoxin was obtained.

Specific rotation: $[\alpha]_D^{17} = -57.2°$ ($CHCl_3$)

(2) Synthesis of 4-O-(2-amino-2-deoxy-4,6-ethylidene-β-D-glucopyranosyl)-4′-demethyl-4-epipodophyllotoxin

(a) 180 mg of 4-O-(2-benzyloxycarbonylamino-2-deoxy-β-D-glucopyranosyl)-4′-benzyloxycarbonyl-4′-demethyl-4-epipodophyllotoxin and 0.5 ml of acetaldehyde diethylacetal were dissolved in 5 ml of acetonitrile. 10 mg of p-toluenesulfonic acid was added to the solution, and the mixture was stirred for 30

minutes at room temperature. Sodium bicarbonate was then added to the mixture, and the insolubles were separated by filtration. The filter cake was washed with dichloromethane, and the washings were combined with the filtrate, followed by concentrating the combined liquid. The concentrate was chromatographed on silica gel for separation and purification. 171 mg of 4-O-(2-benzyloxycarbonylamino-2-deoxy-4,6-ethylidene-β-D-glucopyranosyl)-4'-benzyloxycarbonyl-4'-epipodophyllotoxin was obtained.

Specific rotation: $[\alpha]_D^{18} = -54.1°$ ($CHCl_3$)

(b) 171 mg of the compound obtained in the above step was dissolved in a solvent mixture of 3 ml of ethyl acetate and 2 ml of acetone. 10 mg of palladium black was added to the solution, and the mixture was stirred for 8 hours, with hydrogen blown therethrough, to perform reduction. The reaction mixture was filtered, and the filtrate was concentrated to dryness. Recrystallization from ethyl acetate gave 73 mg of the desired 4-O-(2-amino-2-deoxy-4,6-ethylidene-β-D-glucopyranosyl)-4'-demethyl-4-epipodophyllotoxin. The mother liquor was chromatographed on silica gel for separation and purification. The desired compound was recovered in an amount of 15 mg.

Melting point: 201—215°C
Specific rotation: $[\alpha]_D^{21} = -89.8°$ ($CH_3OH$)
MS (SIMS): 588 $(M + H)^+$
NMR (Pyridine-$d_5$): δ 1.36 (3Hd, $CH_3$), 3.74 (6Hs, $OCH_3$),
5.05 (1Hd, H—4), 5.24 (1Hd, H—1),
5.92 (2Hs, —O—$CH_2$—O—),
6.72 (1Hs, H—8),
6.75 (2Hs, H—2',6')
7.45 (1Hs, H—5)
1764 (C=O)$cm^{-1}$

## Example 2

Synthesis of 4-O-(3-amino-3-deoxy-4,6-O-ethylidene-β-D-glucopyranosyl)-4'-demethyl-4-epipodophyllotoxin

(a) 486 mg of 4'-benzyloxycarbonyl-4'-demethyl-4-epipodophyllotoxin and 360 mg of 2,4,6-tri-O-acetyl-3-benzyloxycarbonylamino-3-deoxy-β-D-glucopyranose were dissolved in 1 ml of dichloromethane. With the solution cooled at −20°C, 0.5 ml of $BF_3 \cdot Et_2O$ was added dropwise over 3 minutes, and the mixture was reacted for 30 minutes, with stirring, under the atmosphere of argon. 0.5 ml of pyridine was added to terminate the reaction, and the reaction mixture was diluted with 20 ml of dichloromethane, then was washed twice with 10 ml of water. The organic layer was dried over Glauber's salt and concentrated. The concentrate was separated and purified by silica gel chromatography to obtain 500 mg of 4 - O - (2,4,6 - tri - O - acetyl - 3 - benzyloxycarbonylamino - 3 - deoxy - β - D - glucopyranosyl) - 4' - benzyloxycarbonyl - 4' - demethyl - 4 - epipodophyllotoxin.

Specific rotation: $[\alpha]_D^{19} = -40.1°$ ($CHCl_3$)

(b) 500 mg of the compound obtained by Step (a) and 100 mg of zinc acetate were dissolved, with heating, in a solvent mixture of 2 ml of methanol and 2 ml of dioxane. The solution was boiled for 6 hours under reflux, and the reaction mixture obtained was concentrated to dryness. 20 ml of dichloromethane and 10 ml of water were added to the residue, and the mixture was shaken vigorously. The organic layer was separated from the system, dried over Glauber's salt, and concentrated. The concentrate was separated and purified by silica gel chromatography to obtain 155 mg of 4-O-(2-O-acetyl-3-benzyloxycarbonylamino-3-deoxy-β-D-glucopyranosyl-4'-benzyloxycarbonyl-4'-demethyl-4-epipodophyllotoxin.

Specific rotation: $[\alpha]_D^{18} = -31.8°$ ($CHCl_3$)

(c) 140 mg of the compound obtained in Step (b) and 0.5 ml of acetaldehyde diethylacetal were dissolved in 3 ml of acetonitrile. 5 mg of p-toluenesulfonic acid was added to the solution, and the mixture was reacted for 2 hours at room temperature with stirring. Sodium bicarbonate was added, and the insolubles were collected by filtration. The filter cake was washed with dichloromethane, and the washings were combined with the filtrate, followed by concentrating the combined liquid. The concentrate was separated and purified by chromatography to obtain 106 mg of 4-O-(2-acetyl-3-benzyloxycarbonylamino-3-deoxy-4,6-O-ethylidene-β-D-glucopyranosyl)-4'-benzyloxycarbonyl-4'-demethyl-4-epipodophyllotoxin.

Specific rotation: $[\alpha]_D^{25} = -35.8°$ ($CHCl_3$)

(d) 100 mg of the compound obtained in Step (c) was dissolved in 3 ml of ethyl acetate, and palladium black was added to the solution. The mixture was stirred for 3 hours in a hydrogen gas steam for reduction. The reaction mixture was filtered and concentrated. The concentrate was separated and purified by silica gel chromatography to obtain 39 mg of 4-O-(2-O-acetyl-3-amino-3-deoxy-4,6-O-ethylidene-β-D-glucopyranosyl)-4'-demethyl-4-epipodophyllotoxin.

NMR ($CDCl_3$): δ 1.35 (3Hd, $CH_3$,) δ 3.87 (3Hs, $CH_3\overset{O}{\overset{\|}{C}}$—),
3.88 (6Hs, —$OCH_3$), 4.81 (1Hd, H—4),
5.97 (2Hs, —$OCH_2O$—), 6.24 (2Hs, H—2',6'),
6.53 (1Hs, H—8), 6.85 (1Hs, H—5)

(e) 29 mg of the compound obtained in Step (d) and 5 mg of zinc acetate were dissolved in 3 ml of methanol, and the solution was boiled for 45 minutes under reflux. The reaction mixture was poured into 10 ml of water, and then extracted twice with 10 ml of dichloromethane. The extract was concentrated, and the concentrate was separated and purified by silica gel chromatography to obtain 7.4 mg of 4-O-(3-amino-3-deoxy-4,6-O-ethylidene-β-D-glucopyranosyl)-4'-demethyl-4-epipodophyllotoxin.

Melting point: 210—220°C

Specific rotation: $[\alpha]_D^{23} = -94.7°$ ($CHCl_3$)

MS (SIMS): 588 $(M + H)^+$

NMR ($CDCl_3$): δ 1.35 (3Hd, $CH_3$),
3.77 (6Hs, $—OCH_3$),
4.90 (1Hd, H—4),
5.97 (2H broads, $—O—CH_2—O—$),
6.27 (2Hs, H—2',6'),
6.55 (1Hs, H—8),
6.85 (1Hs, H—5),

IR: 1765 (c=O) $cm^{-1}$

Reference Example

Synthesis of saccharide

700 mg of 3,4,6-tri-O-acetyl-2-benzyloxycarbonylamino-2-deoxy-β-D-glucopyranosyl bromide [Bull, Chem. Soc. Japn. 34, 183 (1963)] was dissolved in 2 ml of acetone, and the solution was cooled to 0°C. 290 mg of silver carbonate and 20 ml of water were added to the solution and the mixture was stirred for 1 hour at this temperature and filtered. The filtrate was concentrated to obtain 580 mg of 3,4,6-tri-O-acetyl-2-benzyloxycarbonyl-amino-2-deoxy-β-D-glucopyranose. In this Referential Example, 2,4,6-tri-O-acetyl-3-benzyloxycarbonylamino-3-deoxy-β-D-glucopyranosyl bromide can be used as the starting compound and treated in the same way to obtain 2,4,6-tri-O-acetyl-3-benzyloxycarbonylamino-3-deoxy-β-D-glucopyranose instead.

**Claims**

1. A novel 4'-demethyl-4-epipodophyllotoxin derivative of the following general formula

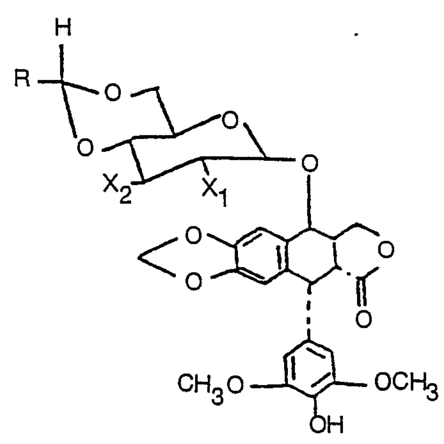

wherein R represents a $C_1—C_4$ alkyl group, $X_1$ represents a hydroxy group or an amino group, and $X_2$ represents an amino group or a hydroxy group, with the proviso that when one of $X_1$ and $X_2$ represents an amino group, the other represents a hydroxy group, and when one of them represents a hydroxy group, the other represents an amino group, and a salt thereof.

2. A compound as claimed in claim 1 for use as a medicament.

3. A process for the preparation of a compound of the formula I

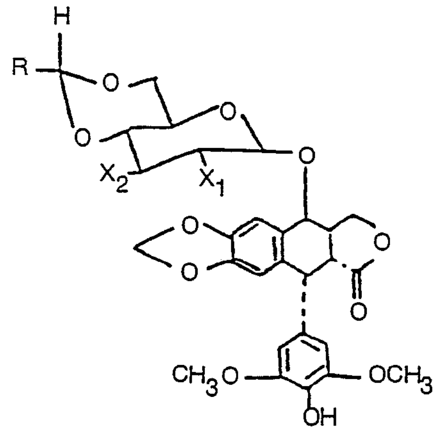

wherein R represents a $C_1$—$C_4$ alkyl group, $X_1$ represents a hydroxy group or an amino group, and $X_2$ represents an amino group or a hydroxy group, with the proviso that when one of $X_1$ and $X_2$ represents an amino group, the other represents a hydroxy group, and when one of them represents a hydroxy group, the other represents an amino group, or a salt thereof, which comprises condensing a compound of the formula V

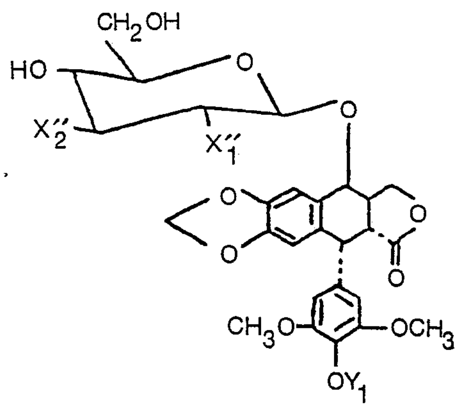

(V)

wherein one of $X''_1$ and $X''_2$ represents a hydroxyl group or a protected hydroxyl group and the other represents a protected amino group, and $Y_1$ represents a protective group, with an aldehyde of the formula VI

RCHO

wherein R is as defined in formula I, or its acetal compound in the presence of a condensing agent, to form a compound of the formula VII

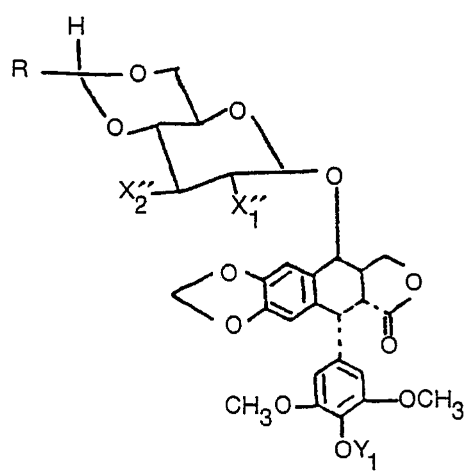

(VII)

wherein R, $X''_1$, $X''_2$ and $Y_1$ are each as defined above, and removing the protective groups therefrom.

**Patentansprüche**

1. Neue 4'-Demethyl-4-epipodophyllotoxin-Derivate der folgenden allgemeinen Formel

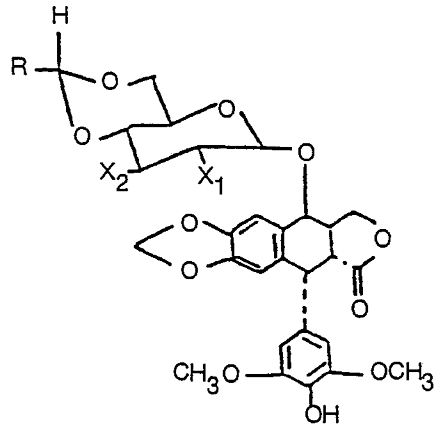

worin R eine $C_1$—$C_4$-Alkylgruppe, $X_1$ eine Hydroxyl- oder eine Aminogruppe, und $X_2$ eine Amino- oder eine Hydroxylgruppe bedeuten, mit der Massgabe, dass, falls einer der beiden, $X_1$ oder $X_2$, eine Aminogruppe bedeutet, der andere eine Hydroxylgruppe bedeutet, und falls einer von ihnen eine Hydroxylgruppe bedeutet, der andere eine Aminogruppe bedeutet, und deren Salze.

2. Verbindungen gemäss Anspruch 1 zur Verwendung als ein Medikament.

3. Verfahren zur Herstellung von Verbindungen der Formel I

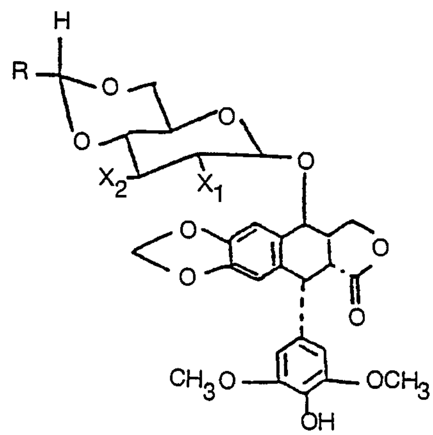

worin R eine $C_1$—$C_4$-Alkylgruppe, $X_1$ eine Hydroxyl- oder eine Aminogruppe, und $X_2$ eine Amino- oder eine Hydroxylgruppe bedeuten, mit der Massgabe, dass, falls einer der beiden, $X_1$ oder $X_2$, eine Aminogruppe bedeutet, der andere eine Hydroxylgruppe bedeutet, und falls einer von ihnen eine Hydroxylgruppe bedeutet, der andere eine Aminogruppe bedeutet, oder eines ihrer Salze, dadurch gekennzeichnet, dass man eine Verbindung der Formel V

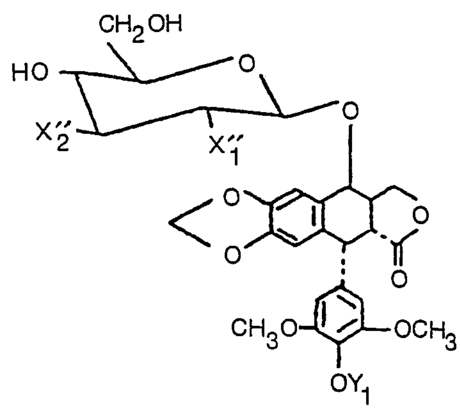

(V)

worin eine der beiden $X''_1$ oder $X''_2$ eine Hydroxylgruppe oder eine geschützte Hydroxylgruppe und die andere eine geschützte Aminogruppe bedeuten, und $Y_1$ eine Schutzgruppe ist, mit einem Aldehyd der Formel VI

RCHO

worin R wie in Formel I definiert ist, oder seiner Acetalverbindung in Anwesenheit eines Kondensationsmittels, kondensiert unter Bildung einer Verbindung der Formel VII

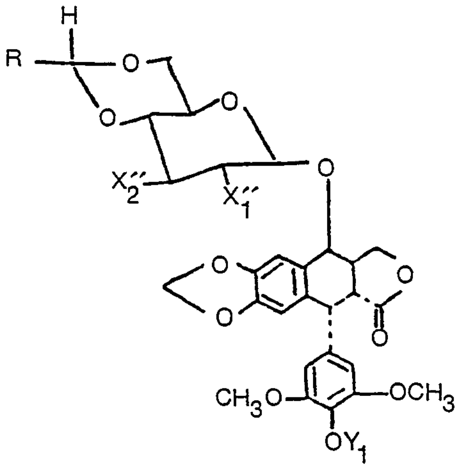

(VII)

worin R, $X''_1$, $X''_2$ und $Y_1$ jeweils wie oben definert sind, und ihre Schutzgruppen abspaltet.

**Revendications**

1. Nouveau dérivé de la 4'-déméthyl-4-épipodophyllotoxine, correspondant à la formule générale suivante:

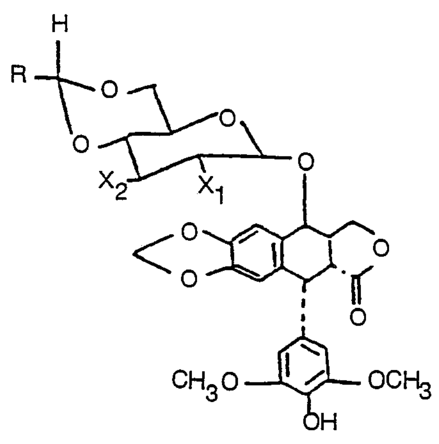

dans laquelle R représente un groupe alkyle en $C_1$—$C_4$, $X_1$ représente un groupe hydroxyle ou un groupe amino, et $X_2$ représente un groupe amino ou un groupe hydroxy à condition que lorsque l'un des groupes $X_1$ et $X_2$, représente un groupe amino, l'autre représente un groupe hydroxy, et que lorsque l'un d'eux représente un groupe hydroxy, l'autre représente un groupe amino; et un sel de celui-ci.

2. Composé suivant la revendication 1, destiné à être utilisé comme médicament.

3. Procédé de préparation d'un composé de formule I:

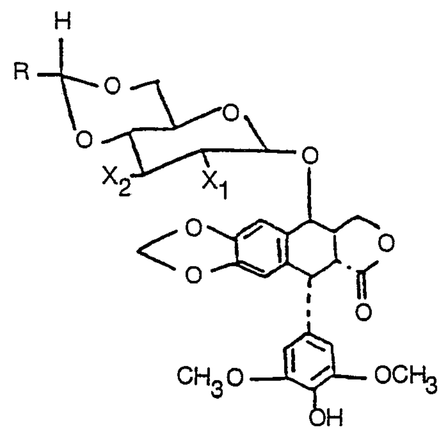

dans laquelle R représente un groupe alkyle en $C_1$—$C_4$, $X_1$ représente un groupe hydroxyle ou un groupe amino, et $X_2$ représente un groupe amino ou un groupe hydroxy à condition que lorsque l'un des groupes $X_1$ et $X_2$, représente un groupe amino, l'autre représente un groupe hydroxy, et que lorsque l'un d'eux représente un groupe hydroxy, l'autre représente un groupe amino; et un sel de celui-ci, dans lequel on condense un composé de formule V:

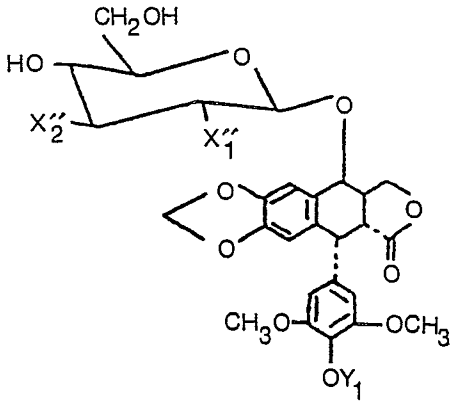

(V)

dans laquelle l'un des groupes $X''_1$ et $X''_2$, représente un groupe hydroxyle ou un groupe hydroxyle protégé, et l'autre représente un groupe amino protégé, et $Y_1$ représente un groupe protecteur, avec un aldéhyde de formule VI

RCHO

dans laquelle R est tel que défini dans la formule I, ou son dérivé correspondant de type acétal en présence d'un agent de condensation pour former un composé de formule VII

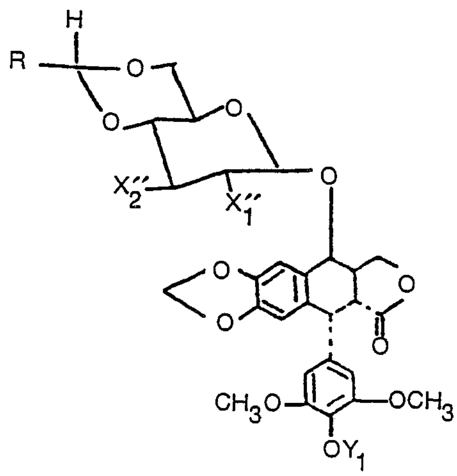

(VII)

dans laquelle les groupes R, $X''_1$, $X''_2$ et $Y_1$, correspondent chacun aux définitions mentionnées ci-dessus, et on élimine les groupes protecteurs de celui-ci.